# EUROPEAN PATENT SPECIFICATION

(11) **EP 2 806 856 B1**
(45) Date of publication and mention of the grant of the patent: **20.04.2016**
(21) Application number: 13704377.4
(22) Date of filing: 25.01.2013
(51) Int. Cl.: A61K 9/16, A61K 31/5685, B02C 19/06

(54) **DRUG/CARRIER INCLUSION COMPOSITES PREPARED BY A MECHANOCHEMICAL ACTIVATION PROCESS USING HIGH-ENERGY FLUID-JET MILLS**
DURCH MECHANOCHEMISCHES AKTIVIERUNGSVERFAHREN ANHAND VON HOCHENERGETISCHEN FLÜSSIGKEITSSTRAHLMÜHLEN HERGESTELLTE ARZNEISTOFF-/TRÄGEREINSCHLUSSVERBUNDSTOFFE
COMPOSITES D'INCLUSION MÉDICAMENT/EXCIPIENT FABRIQUÉS PAR UN PROCÉDÉ D'ACTIVATION MÉCANOCHIMIQUE UTILISANT DES BROYEURS À JET DE FLUIDE À HAUTE ÉNERGIE

(30) Priority: 26.01.2012 IT MI20120092
(43) Date of publication of application: 03.12.2014
(73) Proprietor: Micro-Macinazione S.A., 6995 Molinazzo di Monteggio (CH)
(72) Inventor: CARLI, Fabio, 6995 Molinazzo Di Monteggio (CH); IAMARTINO, Piero, 6995 Molinazzo Di Monteggio (CH); LEONE, Milko, 6995 Molinazzo Di Monteggio (CH)
(74) Representative: Minoja, Fabrizio
(86) International application number: PCT/EP2013/051502
(87) International publication number: WO 2013/110789

(56) References cited:
- EP-A2- 2 286 797
- WO-A1-03/002097
- WO-A2-96/32931
- WO-A2-03/082241
- US-A1- 2008 031 766

## Description

### SUMMARY OF THE INVENTION

Drug/carrier inclusion composites comprising a drug and a carrier material (selected from linear polymers, crosslinked polymers and complexing agents such as cyclodextrins) are prepared by a high-energy co-grinding process using fluid-jet mills, suitably modified to convert the co-grinding process into mechanochemical activation. The activated drug/carrier composites thus obtained, in the case of poorly soluble drugs, present much improved chemico-physical and biopharmaceutical characteristics compared with the drugs "as is": a high percentage of amorphous phase, nanocrystallinity, high solubilisation kinetics and better bioavailability.

In the case of water-soluble drugs, mechanochemical activation with a hydrophobic carrier produces modified release (e.g. slow release).

### INTRODUCTION - PRIOR ART

Approximately 40% of the drugs currently under development are poorly soluble in water (Lipinski, 2002; Merisko-Liversidge & Liversidge, 2008); in other words they belong to Class II of the Biopharmaceutical Classification System (BCS) for APIs (Active Pharmaceutical Ingredients). This class comprises drugs with considerable problems of oral absorption, such as low bioavailability and possibly slow absorption, greater variability between patients, and a potentially greater possibility of effects generated by food intake (Amidon et al., 1995).

A number of formulation and process approaches have been proposed to improve the solubility and bioavailability of poorly soluble, poorly absorbed drugs: preparation of solid dispersions using water-soluble carriers (Craig & Newton, 1992; Weuts et al., 2005; Ahuya et al., 2007), complexing with cyclodextrins (Veiga et al., 2001), application of the supercritical fluid technique (Hussein et al., 2008), spray freezing technology (Rogers T.L. et al., 2002), and development of auto-emulsifying solid systems (Tange et al., 2008).

Micronisation (Chaumeil, 1998), namely a reduction in the particle size of the drug leading to an increase in its surface area, has been widely described as a technique for improving the dissolution rate and biopharmaceutical properties of poorly soluble drugs.

Hydrophilic drug/carrier co-grinding has been repeatedly proposed (Friedrich, Bodmaier, 2005) since the description in 1977 of co-grinding of drugs with microcrystalline cellulose (Chem. Pharm. Bull., 78, 3340-6, 1977) and the use of a vibration mill on mixtures of the drug with the polymer 1-4 glucan (US 4036990). Complexes with beta-cyclodextrin have been obtained with the use of high-energy vibration mills (EP 0449167), inclusion systems based on drugs loaded on silica gel have also been obtained, again using high-energy mills (EP 129893); crosslinked polymers which are insoluble but swell in water, such as crospovidone, were proposed as carriers applying the vibration mill technique in US 4639370; WO 96/32931 proposes crosslinked sodium carboxymethyl starch as carrier for high-energy mills such as centrifugal or vibration mills. US 2005/0255163 describes the use of aminoacids as catalysts of the activation process to obtain better results in shorter co-grinding times using ternary drug/carrier/aminoacid mixtures, but once again the mills used are ball, vibration or centrifugal mills. A technological modification of the activation process was proposed in US 5449521, which disclosed the addition of water vapour or solvent to the co-grinding chamber of vibration mills to promote the destructuring process of the drug crystals.

WO 03/002097 only generically mentions the air jet mill as a potential mill to be used but no concrete experimental example is presented using such mill. In this document in fact all the experimental examples are carried out using an high energy centrifugational mill based on the very strong impact of high density media over the powder mixtures of the drug and carrier, with consequent potential contamination of the resulting activated composite with media material and with grinding chamber lining material. Moreover, this document discloses a process based on the combination of a second step of exposure to water vapor with the first step of co-grinding of drug and carrier.

WO 03/082241 refers to a controlled release formulation of clarithtromycin, where clarithromycin is micronized with an air jet mill to a very fine particle size in the presence of an inert material to prevent aggregation of the drug particles.

There is no reduction in crystallinity of the drug and no inclusion of the drug into the carrier particles: actually a conventional air jet mill which has not been modified to obtain a prolonged time of exposure to high energy impact and consequent amorphization of the drug and inclusion into the carrier.

EP 2286797 describes the co-grinding of a drug and inert additive particles to obtain a powder for pulmonary inhalation. The disclosed process aims at obtaining a very fine particle size of drug in order to increase the fraction deliverable to lungs after inhalation and to optimize the detachment of drug particles from the carrier particles after inhalation. No inclusion of the drug into the carrier particles occurs: on the contrary, inclusion is carefully avoided using lubricating substances to favor the detachment of drug particles from the carrier particles. Consequently, the air jet mill is not modified or optimized (closed system) to induce amorphization or inclusion of the drug into the carrier particles. On the contrary the whole process is optimized by careful selection of particle sizes of starting materials and lubricating properties of additives to avoid inclusion or strong penetration of drug particles onto carrier.

WO 96/32931 discloses the high energy co-grinding of drugs with a single specific carrier material, sodium starch glycolate. The mill described and used in the experimental examples is only the high energy vibration mill, which is based on the high impact of the grinding media over the drug and carrier powders, with consequent potential contamination of the resulting composite drug/carrier composite with material released from the media and the lining of the grinding chamber. No reference is made to the use of an air jet mill to obtain a sufficient energy level to induce amorphization and inclusion of the drug into the carrier.

US 2008/031766 relates to the process of grinding of titanium powders with an air jet mill under inert gas in order to obtain a reduced particle size of the titanium powders with consequent optimal apparent density and no oxygen uptake. No reference is again made to modification of the air jet mill to increase the impact energy in order to obtain the inclusion of the active of interest into a carrier particle. The object disclosed process merely focuses on the modification of the apparent density of titanium powders and not on the inclusion of the titanium into another carrier material. Actually the process describes the grinding of one single material (titanium) and not a co-grinding of mixtures of different materials.

The prior art does not accordingly disclose or suggest how to optimize a drug/carrier co-grinding process by means of an air jet mill modified so as to effectively obtain a high energy impact and a residence time in the grinding chamber sufficient to obtain an inclusion of the drug into the carrier with consequent drug amorphization.

### DISCLOSURE OF THE INVENTION

As reported above, the use of mills operating with jets of air or other gaseous fluids is not among the current approaches to drug/carrier co-grinding processes aimed at improving the biopharmaceutical properties of poorly soluble drugs, because the operating procedure and process parameters used to date have prevented the energy levels necessary to induce molecular dispersion of the drug in the carrier material from being reached.

In fact, in the normal operational methods of micronisation of fluid-jet mills, a grading system connected to the grinding chamber causes the finest powder to exit from the chamber; on reaching the required size, said powder tends to migrate towards the cyclone filter, with consequent expulsion and external collection, thus generating an efficient continuous process. With these operating methods, drug/carrier mixtures co-ground with fluid-jet mills, though better than simple physical drug/carrier mixtures, cannot reach the high-energy activation levels that can be obtained with the alternative mills currently used for this purpose, such as ball mills, vibration mills, centrifugal mills, etc.

It has now surprisingly been discovered that co-grinding drug/carrier mixtures with gaseous fluid-jet mills can for all purposes perform efficient mechanochemical activation leading to considerable amorphisation of the active ingredient and molecular distribution of the drug in the carrier if the gaseous fluid-jet mills are modified in such a way as to make the drug/carrier mixtures to be activated in the chamber, in which the mixture is subjected to the shock action of the expanding mass of fluid, last for long, regulatable periods.

In particular, if a fluid-jet mill is modified in such a way as to allow the powder mixture to undergo the shock effect produced by the expanding fluid over a long period, considerable levels of mechanochemical activation can be reached in times (1-4 hours) which are at least comparable with, and can be even shorter than, those normally obtained with the high-energy mills used to date (for example, in the case of ball grinders, up to 12-24 hours may be required).

The first subject of the invention is therefore a process of preparation of drug/carrier inclusion composites which involves co-grinding the mixture of drug and carrier powders in a fluid-jet mill, in particular one in which the fluid is air or nitrogen, suitably modified to allow mechanical fusion of the powders, whereby said fluid-jet mill comprises a milling chamber, a grader, inlets for powders and for gas under pressure, and is characterised by a recirculation pipe between the outlet of the grader and the milling chamber.

The mill is preferably modified in such a way as to allow the drug/carrier powder mixture to be maintained in the co-grinding chamber for a sufficient time to induce mechanical fusion of the powders.

Said process is applicable to drugs characterised by poor solubility or insolubility in water and to drugs characterised by high solubility in water.

A second subject of the invention is a fluid-jet mill which comprises a grinding chamber, a grader, powder inlets and pressurised gas inlets, and is characterised by a recirculation pipe between the grader outlet and the grinding chamber.

The invention also relates to the products of inclusion obtained from the process.

A considerable advantage of the process according to the invention compared with other high-energy co-grinding techniques is that the drug and carrier particles under activation do not come into contact with components of the mill subjected to high shock/stress energy, or with mechanical parts (the central rotor and the walls of the cylindrical chamber of the Hosokawa system), or with the grinding means (e.g., centrifugal ball mills, vibration mills with high-density cylindrical media), but only remain subject to shocks between them, therefore completely preventing the risks that the powders will be contaminated by residues potentially released from the mechanical parts under shock stress or from the grinding means.

It is therefore evident that eliminating all possible contamination produces better-quality drug/carrier composites than those generated with the high-energy processes used to date, with additional advantages in terms of costs due to the reduction in quality controls.

Another major advantage of the drug/carrier composites obtained by the process according to the invention compared with the high-energy processes according to the prior art is an increase in the co-grinding yield, because it drastically reduces the possibility that the activated product will adhere to the grinding equipment (which is absent in the case of fluid-jet mills) or to the structural parts of the co-grinding chamber which are subjected to intense stresses in the collision with the powders that undergo the activation process. The need for a "pre-process" stage ("priming" of the mill used with the mixture to be activated for a sufficient time to coat the parts subject to shock stress, or the grinding equipment, with a film of powder) before the main activation process is also therefore eliminated.

### DETAILED DESCRIPTION OF THE INVENTION

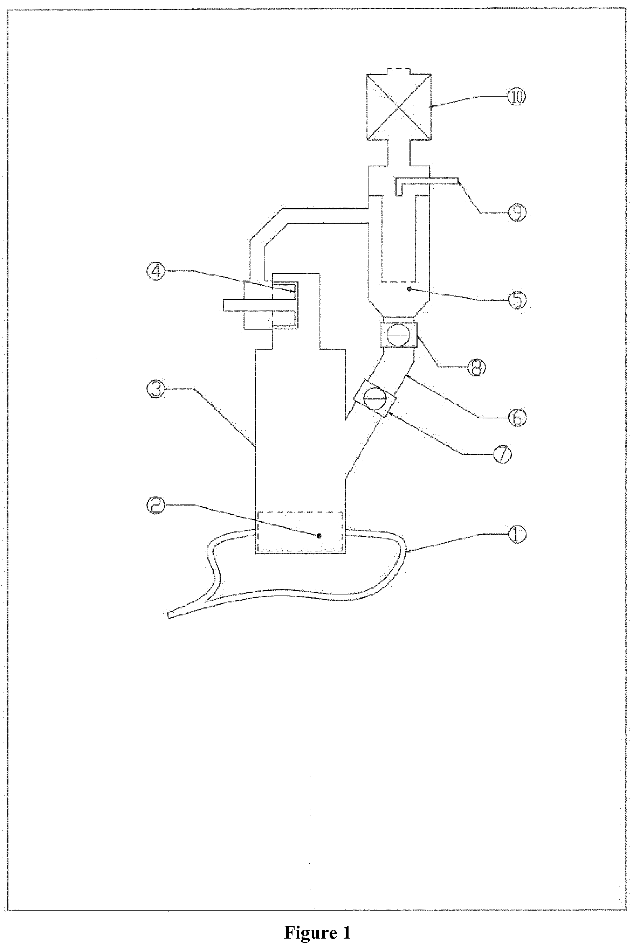

A scheme of a fluid-jet mill modified as described above is shown in figure 1, as one example of the various possible modifications which can be implemented to transform the classic micronisation process into a mechanochemical activation process.

The compressed gases are fed through pipes (1) into co-grinding chamber (2) located in mill chamber (3). The mixture of powders is loaded into the mill and pushed by the flow of the gas through a dynamic grader (4) which only allows particles with a pre-determined size to pass through. Said grading devices are known and can be regulated to predetermine the particle size, for example by varying the rotation speed of the device or the size of the impeller mesh. The particles exiting from the grader can then be reintroduced into grinding chamber (2) through a recirculation system which comprises a separator filter (5) and a pipe (6) communicating with co-grinding chamber (2) via valves (7, 8) which, driven in a suitable sequence, allow the powders to be reintroduced into said co-grinding chamber (2). The separator filter is cleaned with a flow of pressurised gas fed through pipe (9). An absolute filter (10) ensures that the most suitable conditions are maintained in the mill.

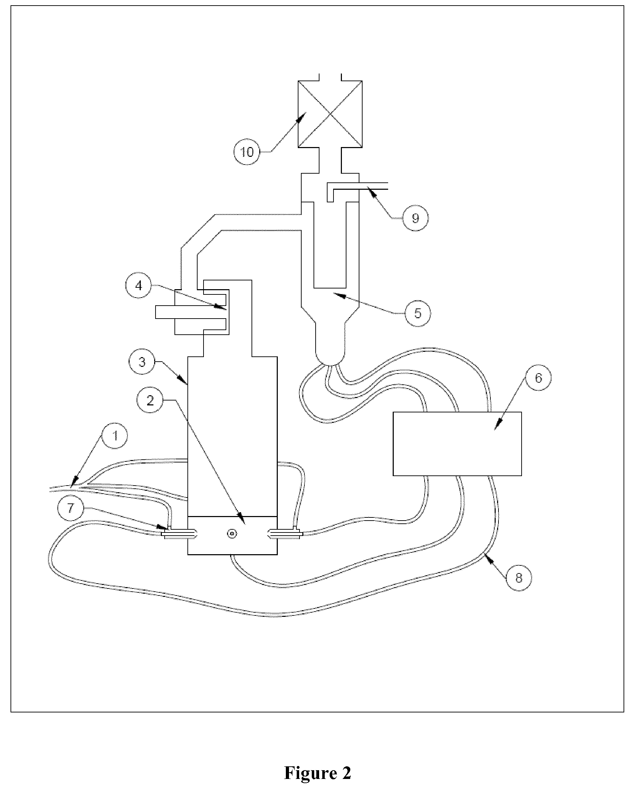

A second example of possible modifications to transform the classic micronisation process into a mechano-chemical activation process is shown in figure 2.

The compressed gases are fed through pipes (1) into the co-grinding chamber (2), by using co-axial nozzles (7) which are also connected to the pipes (8) pumping the powder mixture by using a powder pumping device (6).

The presence of a dynamic grader (4) in the co-grinding chamber allows particles with a pre-determined size to pass through, by varying the rotation speed.

The particles exiting from the grader can then be reintroduced into grinding chamber (2) through a recirculation system which comprises a separator filter (5) and pipelines connecting the bottom part of the separator filter with the powder pumping device (6) which allows a continuous and regular powder transfer.

The separator filter is cleaned with a flow of pressurised gas fed through pipe (9). An absolute filter (10) ensures that the most suitable conditions are maintained in the mill.

The drug/carrier composites obtained with the process according to the invention possess chemico-physical and solubilisation characteristics far superior to those of physical drug/carrier mixtures and to those of co-ground drug/carriers obtained with unmodified fluid-jet mills.

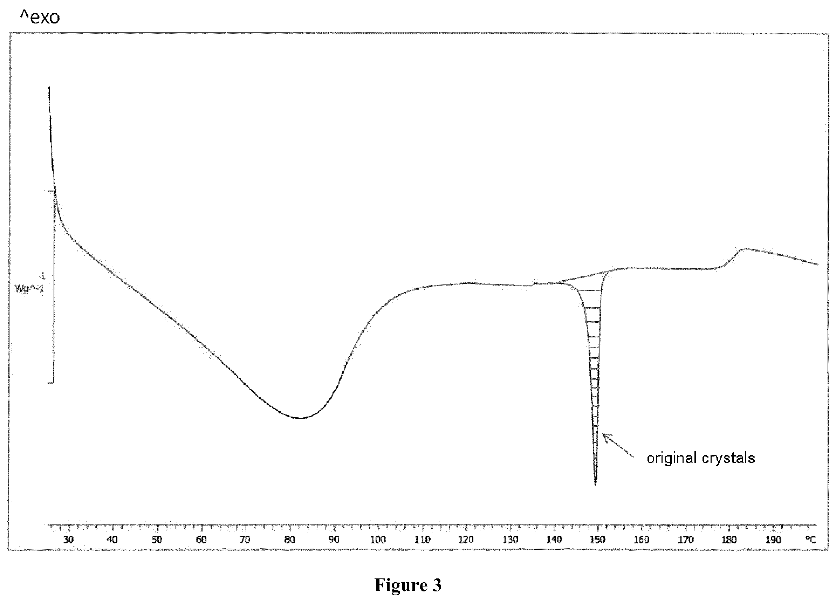

As shown in figures 3-10, the drug/carrier composites according to the invention present a drastically reduced enthalpy of fusion of the drug crystals on DSC (Differential Scanning Calorimetry) analysis (compared with that of the drug as is, see figure 3) as the process proceeds, until practically total amorphisation of the active ingredient is obtained at the end of the process, which in any event is completed in a short time, of approximately 1-3 hours.

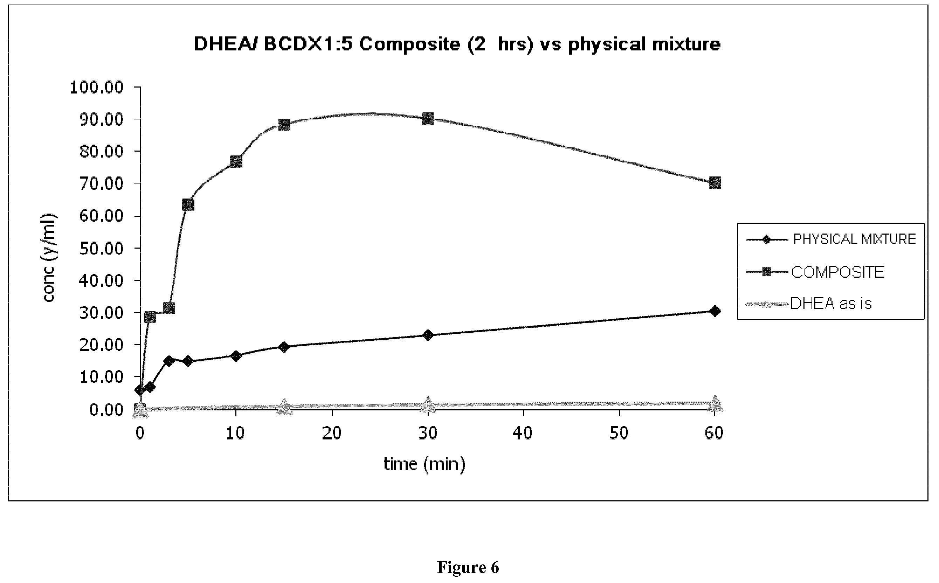

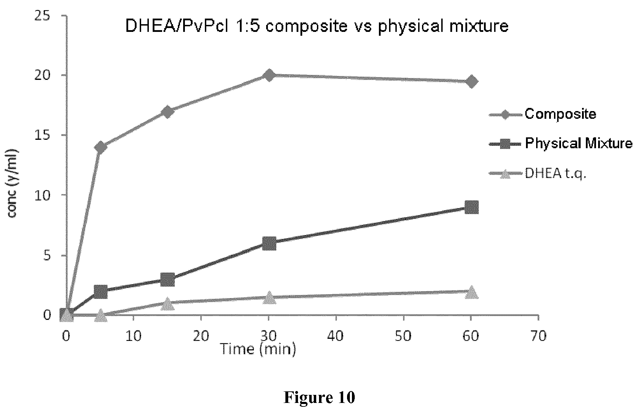

The solubilisation kinetics of the drug/carrier composites according to the invention are also markedly improved compared with those of the corresponding physical drug/carrier mixtures, as shown in figures 6 and 10. The appearance of the solubilisation curve obtained with the composite generated by the process according to the invention is particularly significant (figure 6): it shows supersaturation in a short time followed by a slow reduction in the concentration of the drug in solution, until the final steady-state concentration is reached.

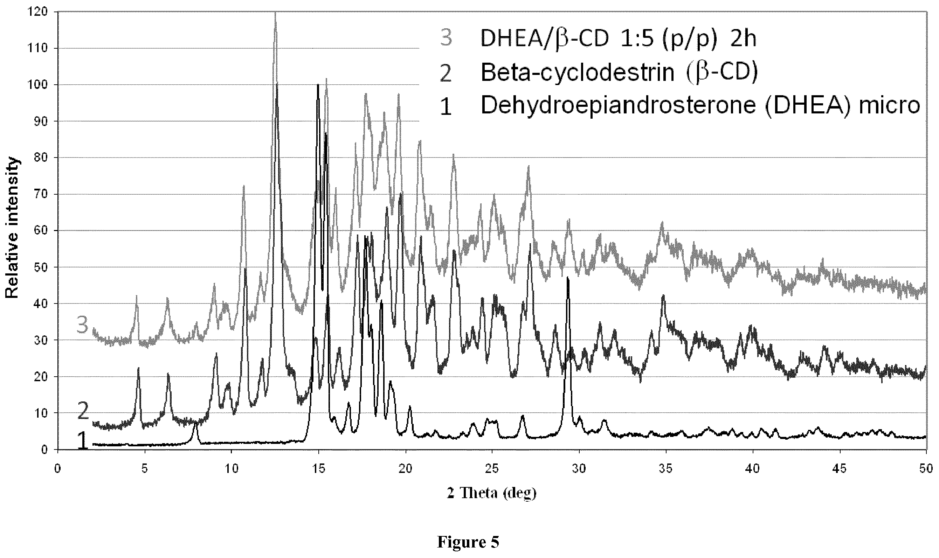

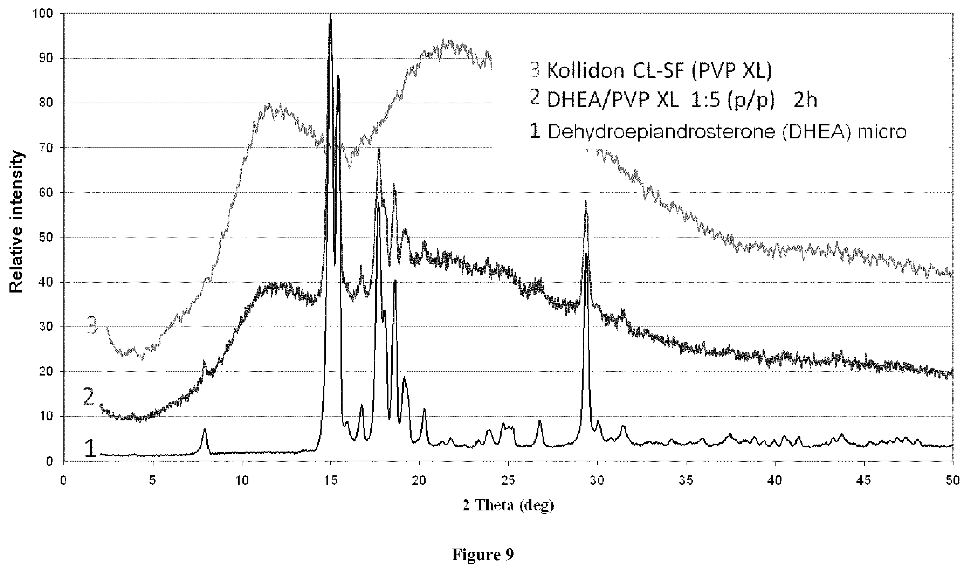

Another surprising aspect of the activated drug/carrier composites prepared by the process according to the invention, compared with the unmodified fluid-jet co-grinding process, is the possibility of obtaining at the end of process, in parallel with the amorphisation of the active ingredient, a reduction in the size of the original crystals still present to nanometre level, namely generation of nanocrystals. The presence of nanocrystals in the activated drug/carrier composites according to the invention is shown by the DSC analyses (see figures 5 and 9): the presence of a melting peak at much lower temperatures than the melting point of the active ingredient can be interpreted as being due to the presence of nanocrystals (as suggested by S. Xiao et al., 2006, K.K. Nanda et al., 2002, and Carli F. et al., 1986). The presence of nanocrystals (and not of polymorph forms) is confirmed by X-ray diffractometry analysis of the same sample (figure 5 and 9), which shows that the residual crystals always have the same internal lattice structure as the original crystals.

Examples of carrier materials which can be used in the process according to the invention include linear polymers such as polyvinylpyrrolidone, hyaluronic acid, chitosan, xanthan, sodium alginate, polyvinyl acetate and sodium starch glycolate; cellulose derivatives, such as sodium carboxymethylcellulose; copolymers such as polyvinylpyrrolidone/polyvinyl acetate, polyvinylpyrrolidone/polyvinyl alcohol, polyvinyl alcohol/PEG, polyvinyl caprolactam/polyvinyl acetate/polyethylene glycol; insoluble polymers that swell in water, such as cross-linked polyvinylpyrrolidone and crosslinked sodium carboxymethylcellulose; complexing agents such as beta-cyclodextrin, alpha-cyclodextrin and hydroxypropyl-beta-cyclodextrin; inorganic materials such as colloidal silicon dioxide, microporous silica gel, polyorganosiloxanes, pharmaceutical clays, barium sulphate, etc.

Numerous poorly soluble drugs can be usefully employed in the activation process according to the invention; non-exhaustive examples are: taxol, ketoprofen, ibuprofen, piroxicam, megestrol acetate, dehydroepiandrosterone, raloxifene hydrochloride, simvastatin, atorvastatin, candesartan, celecoxib, felodipine, budesonide, cisplatin, ritonavir, triazolam, saquinavir, cyclosporin, oestradiol, amiodarone hydrochloride, isradipine, rosiglitazone maleate, etc.

The process according to the invention can also be usefully applied to biotechnological drugs, such as polypeptides, proteins, oligonucleotides and the like, if poorly soluble.

However, if the process according to the invention is applied to water-soluble drugs, if hydrophobic carrier materials are selected, inclusion composites with delayed release and reduced drug dissolution can be obtained, therefore obtaining long-term blood levels with a consequent extension of the therapeutic activity.

Non-exhaustive examples of water-soluble drugs which can be used in the process according to the invention are metoprolol, cimetidine, theophylline, isoniazide, pindolol, propranolol, hydroclorothiazide, atenolol, verapamil, diltiazem, acyclovir, ranitidine, metformin and the like.

Non-exhaustive examples of hydrophobic carrier materials include polymethacrylates, polymethyl methacrylates, ethyl cellulose, acrylate copolymers, ammonium methacrylate copolymers, aminoethyl methacrylate copolymers and hydrophobic silica gel.

The weight ratio between the drug and the carrier is between 1/0.1 w/w and 1/100 w/w, preferably between 1/0.5 and 1/10.

Before being introduced into the co-grinding chamber, the powders can be mixed to increase the homogeneity of distribution of the energy of the expanding fluid on the mass of powder and to promote the generation of the highest possible number of "efficient" collisions between the drug particles and carrier particles, compared with "non-efficient" collisions between the same drug particles or the same particles of carrier material.

The co-grinding time required to achieve good levels of activation, ie. distribution of the active ingredient in the carrier, can vary according to different parameters, such as the weight ratio between drug and carrier (ratios more diluted in favour of the carrier lead to shorter activation times), the pressure value of the expanding fluid (higher pressures usually involve a reduction in co-grinding times), and the desired level of amorphisation or nanocrystallinity. The range of co-grinding times is usually between 0.15 and 24 hours, preferably between 0.5 and 4 hours.

The characteristics of the drug/carrier inclusion composites obtained with the process according to the invention can be determined by one or more of the following methods:
- Differential scanning calorimetry to determine the enthalpy of the residual crystals of the drug, and consequently determine the level of amorphisation reached.
- Differential scanning calorimetry to determine the melting points of the residual crystals, with possible identification of nanocrystals.
- X-ray diffractometry for analysis of the inner structure of the crystalline lattice of any residual crystals, possible leading to the formation of polymorphs.
- Solubilisation kinetics under non-sink conditions in water or saline buffers from 0 to 12-24 hours to determine the improvement in the solubility of the inclusion composites in the steady state compared with the drug as is, and also the rate at which the concentration of the solubilised drug increases, and possible identification of supersaturation associated with significant levels of amorphous phases.

### DESCRIPTION OF FIGURES

**FIGURE 1****:** Diagram showing fluid-jet mill with co-grinding chamber modified to allow long co-grinding times and consequent mechanochemical activation.
**FIGURE 2****:** Diagram showing an alternative configuration of a fluid-jet mill modified to allow co-grinding times and consequent mechanochemical.
**FIGURE 3****:** DSC thermogram of a physical mixture of DHEA/β-CD 1:5 (w/w).
**FIGURE 4****:** DSC thermogram of a DHEA/β-CD 1:5 (w/w) composite after a 2 h mechanochemical activation process.
**FIGURE 5****:** PXRD profiles of DHEA as is, β-CD and a DHEA/β-CD 1:5 (w/w) composite after a 2 h mechanochemical activation process.
**FIGURE 6****:** Solubilisation kinetics (non-sink conditions) in pH 5.5 buffer at 37°C of DHEA as is, a DHEA/β-CD 1:5 (w/w) physical mixture and a DHEA/β-CD 1:5 (w/w) composite after a 2 h mechanochemical activation process.
**FIGURE 7****:** DSC thermogram of a DHEA/Kollidon CL-SF1:3 (w/w) composite after a 2 h mechanochemical activation process.
**FIGURE 8****:** DSC thermogram of a DHEA/Kollidon CL-SF1:5 (w/w) composite after a 2 h mechanochemical activation process.
**FIGURE 9****:** PXRD profiles of DHEA as is, Kollidon CL-SF and a DHEA/Kollidon CL-SF1:5 (w/w) composite after a 2 h mechanochemical activation process.
**FIGURE 10****:** Solubilisation kinetics (non-sink conditions) in pH 5.5 buffer at 37°C of DHEA as is, Kollidon CL-SF and a DHEA/Kollidon CL-SF1:5 (w/w) composite after a 2 h mechanochemical activation process.

Some examples illustrating the process according to the invention and the drug/carrier inclusion composites obtainable are set out below, with the corresponding characterisations.

### EXAMPLE 1

100 g of dehydroepiandrosterone (DHEA) and 300 g of beta-cyclodextrin (KLEPTOSE®) are mixed for 15 minutes in a Turbula® mixer and fed by a flow of air generated by a Venturi valve into the co-grinding chamber of an opposite jet mill, Jetmill Mod JS 100® (MicroMacinazione S.A.), modified according to the invention and illustrated in figure 1.

The co-grinding process is conducted for up to 3 hours, with the following parameters: N₂ pressure: 8 bar, grader rotation speed: 5000 rpm.

Samples of the activated powder are taken at intermediate times and at the end of the process, and analysed by DSC: a gradual reduction in the enthalpy of fusion of DHEA is recorded, until the value of 14.6 J/g is reached after 3 hours, compared with the initial value of DHEA as is, namely 71.9 J/g, corresponding to approx. 30% amorphisation, taking account of dilution with the carrier. Moreover, the melting point of the residual crystals is 149.4°C, which corresponds perfectly to the 150.2°C of DHEA as is, demonstrating the absence of nanocrystals and polymorphs.

### EXAMPLE 2

120 g of dehydroepiandrosterone (DHEA) and 600 g of beta-cyclodextrin (KLEPTOSE®) are mixed for 15 minutes in a Turbula® mixer and fed by a flow of air generated by a Venturi valve into the co-grinding chamber of an opposite jet mill, Jetmill Mod JS 300® (MicroMacinazione S.A.), modified according to the invention and illustrated in figure 1.

The co-grinding process is conducted for up to 2 hours, with the following parameters: N₂ pressure: 8 bar, grader rotation speed: 5000 rpm.

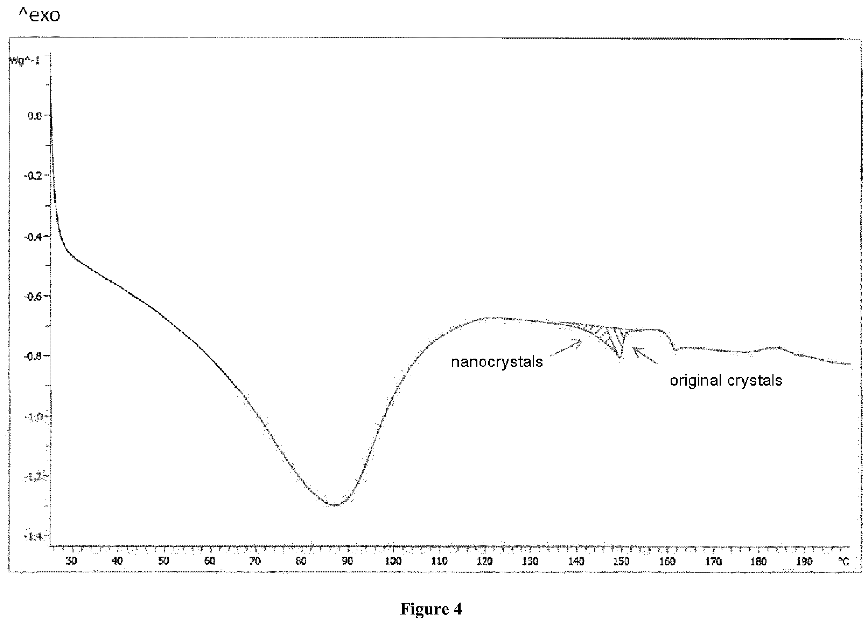

Samples of the activated powder are taken at intermediate times and at the end of the process, and analysed by DSC: a gradual reduction in the enthalpy of fusion of DHEA is recorded, until the value of 2.1 J/g is reached after 2 hours for the sample collected, compared with the initial value of DHEA as is, namely 71.9 J/g, corresponding to approx. 80% amorphisation, taking account of dilution with the carrier (see figure 4).

The melting peak of the residual crystals is 149.2°C, with a tail up to 140°C. The attribution of the lowest melting points to the presence of nanocrystals and not polymorphs is supported by X-ray diffractometry analysis (figure 5), which demonstrates that the inner crystalline structure is identical to that of the original DHEA crystals and that there is less diffraction intensity, confirming the high percentage of amorphisation.

Figure 6 shows the solubilisation kinetics data in a buffer at pH 5.5, 37°C (non-sink conditions generated by the use of a large excess of sample) of the composite after 2 hours' activation; by way of comparison, the same figure shows the data relating to a physical mixture of DHEA/cyclodextrin and to DHEA as is: this shows a great increase (4-5 x) in the concentrations in solution for the activated composite compared with the physical mixture, and an even greater increase compared with the active ingredient as is.

### EXAMPLE 3

100 g of dehydroepiandrosterone (DHEA) and 300 g of crosslinked polyvinylpyrrolidone PVPcl (KOLLIDON CL SF®) are mixed for 15 minutes in a Turbula® mixer and fed by a flow of air generated by a Venturi valve into the co-grinding chamber of an opposite jet mill, Jetmill Mod JS 100® (MicroMacinazione S.A.), modified according to the invention and illustrated in figure 1.

The co-grinding process is conducted for up to 2 hours, with the following parameters: N₂ pressure: 8 bar, grader rotation speed: 5000 rpm.

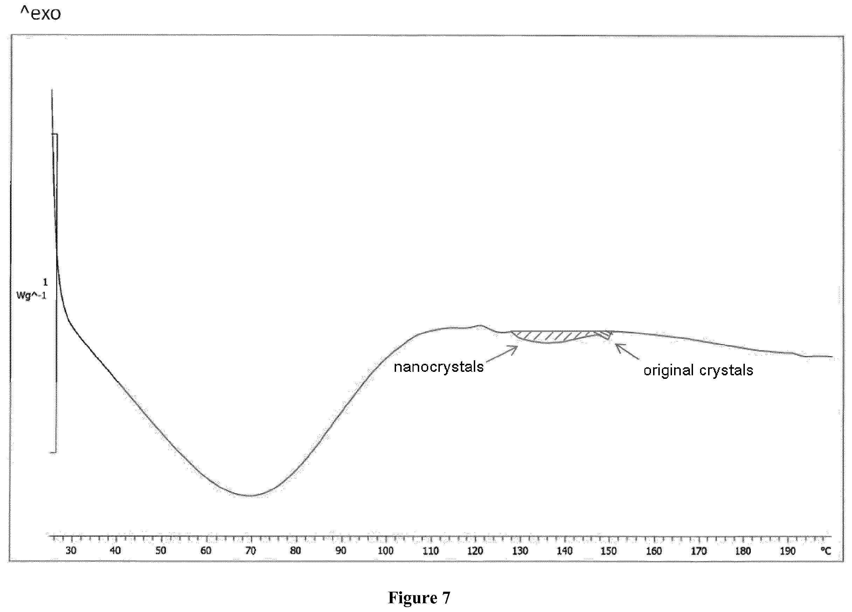

Samples of the activated powder are taken at intermediate times and at the end of the process, and analysed by DSC: a gradual reduction in the enthalpy of fusion of DHEA is recorded until the value of 7.2 J/g is reached after 2 hours for the sample collected, compared with the initial value of DHEA as is, namely 71.9 J/g, corresponding to approx. 80% amorphisation, taking account of dilution with the carrier (see figure 7). By far the highest percentage of the crystalline residue (approx. 80%) is due to nanocrystals (melting points of between 125 and 135°C), and only part (approx. 20%) to original crystals with a melting point of 149.5°C.

### EXAMPLE 4

120 g of dehydroepiandrosterone (DHEA) and 600 g of crosslinked polyvinylpyrrolidone PVPcl (KOLLIDON CL SF®) are mixed for 15 minutes in a Turbula® mixer and fed by a flow of air generated by a Venturi valve into the co-grinding chamber of an opposite jet mill, Jetmill Mod JS 100® (MicroMacinazione S.A.), modified according to the invention and illustrated in figure 1.

The co-grinding process is conducted for up to 2 hours, with the following parameters: N₂ pressure: 8 bar, grader rotation speed: 5000 rpm.

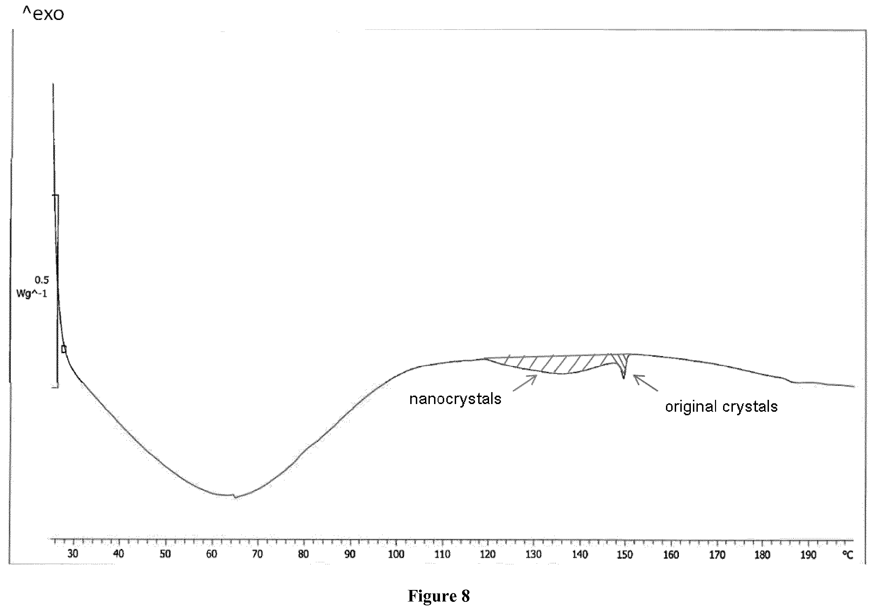

Samples of the activated powder are taken at intermediate times and at the end of the process, and analysed by DSC: a gradual reduction in the enthalpy of fusion of DHEA is recorded, until the value of 1.71 J/g is reached after 2 hours for the sample collected, compared with the initial value of DHEA as is, namely 8.5 J/g, corresponding to approx. 90% amorphisation, taking account of dilution with the carrier (see figure 8). By far the highest percentage of the crystalline residue (approx. 86%) is due to nanocrystals (melting points of between 120 and 145°C), and only part (approx. 14%) to original crystals with a melting point of 149.6°C.

The attribution of the lowest melting points to the presence of nanocrystals and not polymorphs is supported by X-ray diffractometry analysis (figure 9), which demonstrates that the inner crystalline structure is identical to that of the original DHEA crystals and that there is less diffraction intensity, confirming the high percentage of amorphisation.

Figure 10 shows the solubilisation kinetics data of the DHEA/PVPcl 1/5 composite in a buffer at pH 5.5, 37°C (non-sink conditions generated by the use of a large excess of sample), after 2 hours' activation; the same figure shows, by way of comparison, the data relating to a physical mixture of DHEA/PVPcl 1/5 and to DHEA as is: this demonstrates a large relative increase (2-3 x) in the concentrations in solution of the activated composite compared with the physical mixture, and an even greater increase compared with DHEA as is, again indicating the extremely favourable effect on the process whereby the activated state reached by DHEA passes into solution (nanocrystals and molecular dispersion).

### REFERENCES

1. Lipinski C., Am. Pharm. Rev., 5, 82-85, 2002
2. Merisko-Liveridge E.M. et al., Eur. J.Pharm.Sci., 18, 113-120, 2003
3. Amidon G.L. et al., Pharm.Res.,12, 413-420, 1995
4. Craig D. & Newton J.M., Int. J.Pharm.,78, 175-182, 1992
5. Weuts I. et al, Eur. J. Pharm.Sci.,25, 387-393, 2005
6. Ahuja N. et al., Eur. J. Pharm.Biopharm.,65, 26-38, 2007
7. Fernandes C. et al., Eur. J. Pharm. Sci., 15, 79-88, 2002
8. Hussein K. et al., Eur. J. Pharm. Sci., 33, 306-312, 2008
9. Rogers T.L. et al., Pharm. Res., 20, 485-493, 2003
10. Tange B. et al., Drug Discovery Today, 13, 606-612, 2008
11. Chaumeil J.C., Exp. Clin. Pharmacol., 20, 211-215, 1998
12. Friedrich H. et al., DrugDev. Ind. Pharm., 31, 719-728, 2005
13. Chem. Pharm. Bull., 78, 3340-6, 1977
14. US 4036990, Yoshinobu N. et al., 1977
15. EP 0449167, Carli F. & Chiesi P., 1991
16. EP 129893, Masazak M. et al.,1985
17. US 4639370, Carli F., 1987
18. WO 96/32931, Carli et al., 1996
19. US 2005/0255163, Carli F. et al., 2005
20. US 5449521, Lovrecich M., 1995
21. Xiao S. et al., J.Chem.Phys., 125, 184504, 2006
22. Nanda K.K. et al., Phys. Rev., 013208, 2002
23. Carli F. et al., Proceed. 13th Contr. Rel. Symp., Norfolk, USA, 1986

## Claims

1. A process for the preparation of drug/carrier inclusion composites which comprises subjecting the mixture of drug and carrier powders to co-grinding in a fluid-jet mill, suitably modified to allow the mechanical fusion of the powders, whereby said fluid-jet mill comprises a milling chamber, a grader, inlets for powders and for gas under pressure, and is **characterised by** a recirculation pipe between the outlet of the grader and the milling chamber.

2. A process according to claim 1 wherein the mill is modified so as to allow the drug/carrier powder mixture to remain in the co-grinding chamber for a sufficient time to induce mechanochemical activation of the powders.

3. A process according to claim 1 or 2 in which the drug is **characterised by** poor or no solubility in water.

4. A process according to claim 3 in which the drug is selected from taxol, ketoprofen, ibuprofen, piroxicam, megestrol acetate, dehydroepiandrosterone, raloxifene hydrochloride, simvastatin, atorvastatin, candesartan, celecoxib, felodipine, budesonide, cisplatin, ritonavir, triazolam, saquinavir, cyclosporin, oestradiol, amiodarone hydrochloride, isradipine and rosiglitazone maleate.

5. A process according to one or more of claims 1-4 in which the carrier is selected from polyvinylpyrrolidone, hyaluronic acid, chitosan, xanthan, sodium alginate, polyvinyl acetate, sodium starch glycolate, sodium carboxymethylcellulose, polyvinylpyrrolidone/polyvinyl acetate, polyvinyl-caprolactam/polyvinyl acetate/polyethylene glycol, polyvinylpyrrolidone/ polyvinyl alcohol, polyvinyl alcohol/polyethylene glycol, cross-linked polyvinylpyrrolidone, cross-linked sodium carboxymethylcellulose, beta-cyclodextrin, alpha-cyclodextrin, hydroxypropyl-beta-cyclodextrin, microporous silica gel, polyorganosiloxanes, pharmaceutical clays, barium sulfate.

6. A process according to claim 1 or 2 in which the medicament is **characterised by** high water solubility.

7. A process according to claim 6 in which the medicament is selected from metoprolol, cimetidine, theophylline, isoniazide, pindolol, propranolol, hydrochlorothiazide, atenolol, verapamil, diltiazem, acyclovir, ranitidine, metformin.

8. A process according to claim 6 or 7 in which the carrier is selected from polymethacrylates, polymethylmethacrylates, methylcellulose, acrylate copolymers, ammonium methacrylate copolymers, aminoethylmethacrylate copolymers, hydrophobic silica gel.

9. A process according to one or more of the above claims in which the weight ratio between medicament and carrier ranges from 1/0.1 w/w to 1/100 w/w.

10. A jet mill for use in the process of claims 1 to 9 which comprises a milling chamber, a grader, inlets for powders and for gas under pressure, and is **characterised by** a recirculation pipe between the outlet of the grader and the milling chamber.

11. An inclusion product obtained by the process of claims 1-9.

12. A product according to claim 11 in which the weight ratio between medicament and carrier ranges from 1/0.1 w/w to 1/100 w/w.

## Patentansprüche

1. Verfahren zur Herstellung von Wirkstoff/Träger-Einschlusskompositen, umfassend das gemeinsame Mahlen des Gemischs aus Wirkstoff- und Trägerpulver in einer Strahlmühle, die in geeigneter Weise modifiziert ist, um die mechanische Fusion der Pulver zu ermöglichen, wobei die Strahlmühle eine Mahlkammer, einen Grader, Einlässe für Pulver und für Gas unter Druck umfasst und durch ein Rückführungsrohr zwischen dem Auslass des Graders und der Mahlkammer gekennzeichnet ist.

2. Verfahren gemäß Anspruch 1, wobei die Mühle so modifiziert ist, dass das Wirkstoff/Träger-Pulvergemisch ausreichend lange in der gemeinsamen Mahlkammer bleiben kann, um eine mechanochemische Aktivierung der Pulver zu induzieren.

3. Verfahren gemäß Anspruch 1 oder 2, wobei der Wirkstoff durch schlechte Löslichkeit oder Unlöslichkeit in Wasser gekennzeichnet ist.

4. Verfahren gemäß Anspruch 3, wobei der Wirkstoff aus Taxol, Ketoprofen, Ibuprofen, Piroxicam, Megestrolacetat, Dehydroepiandrosteron, Raloxifen-Hydrochlorid, Simvastatin, Atorvastatin, Candesartan, Celecoxib, Felodipin, Budenosid, Cisplatin, Ritonavir, Triazolam, Saquinavir, Cyclosporin, Estradiol, Amiodaron-Hydrochlorid, Isradipin und Rosiglitazonmaleat ausgewählt ist.

5. Verfahren gemäß einem oder mehreren der Ansprüche 1 bis 4, wobei der Träger aus Polyvinylpyrrolidon, Hyaluronsäure, Chitosan, Xanthan, Natriumalginat, Polyvinylacetat, Natriumstärkeglycolat, Natriumcarboxymethylcellulose, Polyvinylpyrrolidon/Polyvinylacetat, Polyvinylcaprolactam/ Polyvinylacetat/Polyethylenglycol, Polyvinylpyrrolidon/Polyvinylalkohol, Polyvinylalkohol/Polyethylenglycol, vernetztem Polyvinylpyrrolidon, vernetzter Natriumcarboxymethylcellulose, beta-Cyclodextrin, alpha-Cyclodextrin, Hydroxypropyl-beta-cyclodextrin, mikroporösem Silicagel, Polyorganosiloxanen, pharmazeutischen Tonen und Bariumsulfat ausgewählt ist.

6. Verfahren gemäß Anspruch 1 oder 2, wobei das Medikament durch eine hohe Wasserlöslichkeit gekennzeichnet ist.

7. Verfahren gemäß Anspruch 6, wobei das Medikament aus Metoprolol, Cimetidin, Theophyllin, Isoniazid, Pindolol, Propranolol, Hydrochlorothiazid, Atenolol, Verapamil, Diltiazem, Acyclovir, Ranitidin und Metformin ausgewählt ist.

8. Verfahren gemäß Anspruch 6 oder 7, wobei der Träger aus Polymethacrylaten, Polymethylmethacrylaten, Methylcellulose, Acrylat-Copolymeren, Ammoniummethacrylat-Copolymeren, Aminoethylmethacrylat-Copolymeren und hydrophobem Silicagel ausgewählt ist.

9. Verfahren gemäß einem oder mehreren der obigen Ansprüche, wobei das Gewichtsverhältnis zwischen Medikament und Träger im Bereich von 1/0,1 bis 1/100 liegt.

10. Strahlmühle zur Verwendung in dem Verfahren gemäß den Ansprüchen 1 bis 9, die eine Mahlkammer, einen Grader, Einlässe für Pulver und für Gas unter Druck umfasst und durch ein Rückführungsrohr zwischen dem Auslass des Graders und der Mahlkammer gekennzeichnet ist.

11. Einschlussprodukt, das nach dem Verfahren gemäß den Ansprüchen 1 bis 9 erhalten ist.

12. Produkt gemäß Anspruch 11, wobei das Gewichtsverhältnis zwischen Medikament und Träger im Bereich von 1/0,1 bis 1/100 liegt.

## Revendications

1. Procédé pour la préparation de composites d'inclusion médicament/support, qui comprend le fait de soumettre le mélange de poudres de médicament et de support à un broyage conjoint dans un broyeur à jet de fluide, modifié de manière appropriée pour permettre la fusion mécanique des poudres, ledit broyeur à jet de fluide comprenant une chambre de broyage, un dispositif de calibrage, des entrées pour les poudres et pour du gaz sous pression, et **caractérisé par** un conduit de recirculation entre la sortie du dispositif de calibrage et la chambre de broyage.

2. Procédé selon la revendication 1, dans lequel le broyeur est modifié pour permettre au mélange de poudres de médicament/support de rester dans la chambre de broyage conjoint pendant un laps de temps suffisant pour induire une activation mécanochimique des poudres.

3. Procédé selon la revendication 1 ou 2, dans lequel le médicament est **caractérisé par** une solubilité médiocre ou une absence de solubilité dans l'eau.

4. Procédé selon la revendication 3, dans lequel le médicament est choisi parmi le taxol, le cétoprofène, l'ibuprofène, le piroxicam, l'acétate de mégestrol, la déshydroépiandrostérone, le chlorhydrate de raloxifène, la simvastatine, l'atorvastatine, le candésartan, le célécoxib, la félodipine, le budésonide, le cisplatine, le ritonavir, le triazolam, le saquinavir, la cyclosporine, l'oestradiol, le chlorhydrate d'amiodarone, l'isradipine et le maléate de rosiglitazone.

5. Procédé selon une ou plusieurs des revendications 1 à 4, dans lequel le support est choisi parmi la polyvinylpyrrolidone, l'acide hyaluronique, le chitosane, le xanthane, l'alginate de sodium, l'acétate de polyvinyle, le glycolate d'amidon de sodium, la carboxyméthylcellulose de sodium, un support de polyvinylpyrrolidone/acétate de polyvinyle, de polyvinylcaprolactame/acétate de polyvinyle/polyéthylène glycol, de polyvinylpyrrolidone/alcool polyvinylique, d'alcool polyvinylique/polyéthylène glycol, la polyvinylpyrrolidone réticulée, la carboxyméthylcellulose de sodium réticulée, la bêta-cyclodextrine, l'alphacyclodextrine, un support d'hydroxypropyl-bêta-cyclodextrine, du gel de silice microporeux, des polyorganosiloxanes, des argiles pharmaceutiques, du sulfate de baryum.

6. Procédé selon la revendication 1 ou 2, dans lequel le médicament est **caractérisé par** une solubilité élevée dans l'eau.

7. Procédé selon la revendication 6, dans lequel le médicament est choisi parmi le métoprolol, la cimétidine, la théophylline, l'isoniazide, le pindolol, le propranolol, l'hydrochlorothiazide, l'aténolol, le vérapamil, le diltiazem, l'acyclovir, la ranitidine, la metformine.

8. Procédé selon la revendication 6 ou 7, dans lequel le support est choisi parmi des polyméthacrylates, des polyméthacrylates de méthyle, la méthylcellulose, des copolymères d'acrylate, des copolymères de méthacrylate d'ammonium, des copolymères de méthacrylate d'aminoéthyle, du gel de silice hydrophobe.

9. Procédé selon une ou plusieurs des revendications ci-dessus, dans lequel le rapport pondéral entre le médicament et le support se situe dans la plage de 1/0,1 en poids/poids à 1/100 en poids/poids.

10. Broyeur à jet pour son utilisation dans le procédé selon les revendications 1 à 9, qui comprend une chambre de broyage, un dispositif de calibrage, des entrées pour les poudres et pour du gaz sous pression et qui est **caractérisé par** un conduit de recirculation entre la sortie du dispositif de calibrage et la chambre de broyage.

11. Produit d'inclusion que l'on obtient via le procédé selon les revendications 1 à 9.

12. Produit selon la revendication 11, dans lequel le rapport pondéral entre le médicament et le support se situe dans la plage de 1/0,1 en poids/poids à 1/100 en poids/poids.
